Europäisches Patentamt

European Patent Office

Office européen des brevets

(19)

(11) **EP 1 304 323 A1**

(12) **EUROPEAN PATENT APPLICATION**
published in accordance with Art. 158(3) EPC

(43) Date of publication:
  **23.04.2003 Bulletin 2003/17**

(51) Int Cl.⁷: **C07D 207/16**, A61K 31/401, A61P 43/00, A61P 17/00, A61K 7/00, A61K 7/48

(21) Application number: **01951930.5**

(22) Date of filing: **19.07.2001**

(86) International application number:
  **PCT/JP01/06269**

(87) International publication number:
  **WO 02/006225 (24.01.2002 Gazette 2002/04)**

(84) Designated Contracting States:
  **AT BE CH CY DE DK ES FI FR GB GR IE IT LI LU MC NL PT SE TR**
  Designated Extension States:
  **AL LT LV MK RO SI**

(30) Priority: **19.07.2000 JP 2000218184**
       **05.09.2000 JP 2000269349**

(71) Applicant: **KYOWA HAKKO KOGYO CO., LTD.**
  **Chiyoda-ku, Tokyo 100-8185 (JP)**

(72) Inventors:
 • **KOBAYASHI, Asako,**
  **Kyowa Hakko Kogyo Co., Ltd**
  **Tsukuba-shi, Ibaraki 305-0841 (JP)**
 • **TAKAHASHI, Tomoya,**
  **Kyowa Hakko Kogyo Co., Ltd**
  **Tsukuba-shi, Ibaraki 305-0841 (JP)**
 • **TAKEKOSHI, Yoichiro**
  **Rye, NY 10580 (US)**

(74) Representative: **VOSSIUS & PARTNER**
  **Siebertstrasse 4**
  **81675 München (DE)**

(54) **PREVENTIVES OR REMEDIES FOR ATOPIC DERMATITIS**

(57)    The present invention provides skin epidermal ceramide synthesis accelerators, agents for improving skin epidermal barrier function and agents for preventing or improving atopic dermatitis, which each comprises, as an active ingredient, hydroxyproline or an *N*-acyl derivative of hydroxyproline, or a salt thereof, and cosmetics for improving skin barrier function or improving atopic dermatitis, which comprises the ceramide synthesis accelerator.

**Description**

TECHNICAL FIELD

The present invention relates to ceramide synthesis accelerators and cosmetics comprising the ceramide synthesis accelerator.

BACKGROUND OF THE INVENTION

The skin is always exposed to stimuli from various external environments. A stratum corneum of the skin has a barrier function to prevent the stimuli and invasion of foreign matters from the outside and to prevent transpiration of moisture from the body. It is known that the amount of ceramides in the stratum corneum of the skin is decreased in human and animals having impaired barrier function [*Journal of Lipid Research*, 30, 89 (1989)]. In addition, it has been reported that the amount of ceramides is also decreased in patients of atopic dermatitis having impaired barrier function [*Acta Dermato Venereologica*, 78, 27 (1998)].

Currently, external use of ceramide has been examined as a means for improving the barrier function, and its efficacy has been confirmed [*Fragrance Journal*, 10, 29 (1999)]. Also, since it is not easy to obtain a large amount of ceramides, an attempt has been made recently to screen a substance capable of increasing ceramide production in the skin.

It is known that there are various kinds of skin ceramides [*Journal of Lipid Research,* 24, 559 (1983)]. It is known that each ceramide has a specific physiological activity. Particularly, it has been reported that the ceramide 1 controls the amount of moisture transpiration by stably keeping intercellular lipids of the stratum corneum and the ceramide 2 contributes to the moisture keeping function of the skin [*Fragrance Journal,* 10, 65 (1999)].

As substances which enhance biosynthesis of ceramides, eucalyptus extract [*The 24th Annual Meeting of the Japanese Society for Investigative Dermatology* (1999)], nicotinic acid (Japanese Published Unexamined Patent Application No. 217658/96), *N*-acetyl-L-cysteine (Japanese Published Unexamined Patent Application No. 291851/95), yeast extract (Japanese Published Unexamined Patent Application No. 2952/97), lactic acid [*Archives of Dermatological Research,* 383-390, 288 (1996)] and the like have been reported.

However, up to now, the activity of accelerating synthesis of the skin epidermal ceramides by hydroxyproline or *N*-acyl derivatives of hydroxyproline has not been known.

DISCLOSURE OF THE INVENTION

An object of the present invention is to provide safe ceramide synthesis accelerators, which accelerate ceramide synthesis in the skin epidermal stratum corneum, improve the skin barrier function and have effects to improve chapped skin or to prevent or improve skin diseases such as atopic dermatitis, and cosmetics comprising the ceramide synthesis accelerator.

The present inventors have conducted intensive studies on the acceleration of ceramide synthesis in the skin epidermal stratum corneum and have found, as a result, that hydroxyproline or N-acyl derivatives of hydroxyproline, or salts thereof have an effect to accelerate ceramide synthesis in the skin epidermal stratum corneum. Thus, the present invention has been accomplished.

That is, the present invention relates to the following (1) to (19).

(1) A skin epidermal ceramide synthesis accelerator which comprises, as an active ingredient, hydroxyproline or an N-acyl derivative of hydroxyproline, or a salt thereof.

(2) The skin epidermal ceramide synthesis accelerator according to (1), wherein the hydroxyproline or the N-acyl derivative of hydroxyproline, or the salt thereof is contained in an amount of 0.01 to 20% by weight based on the total weight.

(3) The skin epidermal ceramide synthesis accelerator according to (1) or (2), wherein the *N*-acyl derivative of hydroxyproline is an *N*-acetylated derivative, an N-propionylated derivative, an *N*-butyrylated derivative or an isobutyrylated derivative of hydroxyproline.

(4) The skin epidermal ceramide synthesis accelerator according to any one of (1) to (3), wherein the hydroxyproline or hydroxyproline in the *N*-acyl derivative of hydroxyproline is an *N*-acyl derivative of hydroxyproline selected from the group consisting of *cis*-4-hydroxy-L-proline, *cis*-4-hydroxy-D-proline, *cis*-3-hydroxy-L-proline, *cis*-3-hydroxy-D-proline, *trans*-4-hydroxy-L-proline, *trans*-4-hydroxy-D-proline, *trans*-3-hydroxy-L-proline and *trans*-3-hydroxy-D-proline.

(5) The skin epidermal ceramide synthesis accelerator according to any one of (1) to (4), wherein the hydroxyproline or hydroxyproline in the N-acyl derivative of hydroxyproline is hydroxyproline produced by a microorganism.

(6) The skin epidermal ceramide synthesis accelerator according to (5), wherein the microorganism is a microorganism into which a proline 3-hydroxylase gene or a proline 4-hydroxylase gene derived from a microorganism belonging to a genus selected from the genus *Amycolatopsis,* the genus *Dactylosporangium* and the genus *Streptomyces* is introduced.

(7) An agent for improving skin epidermal barrier function which comprises, as an active ingredient, hydroxyproline or an *N*-acyl derivative of hydroxyproline, or a salt thereof.

(8) The agent for improving skin epidermal barrier function according to (7), wherein the hydroxyproline or the N-acyl derivative of hydroxyproline, or the salt thereof is contained in an amount of 0.01 to 20% by weight based on the total weight.

(9) The agent for improving skin epidermal barrier function according to (7) or (8), wherein the *N*-acyl derivative of hydroxyproline is an *N*-acetylated derivative, an N-propionylated derivative, an *N*-butyrylated derivative or an isobutyrylated derivative of hydroxyproline.

(10) The agent for improving skin epidermal barrier function according to any one of (7) to (9), wherein the hydroxyproline or the hydroxyproline in the *N*-acyl derivative of hydroxyproline is an *N*-acyl derivative of hydroxyproline selected from the group consisting of *cis*-4-hydroxy-L-proline, *cis*-4-hydroxy-D-proline, *cis*-3-hydroxy-L-proline, *cis*-3-hydroxy-D-proline, *trans*-4-hydroxy-L-proline, *trans*-4-hydroxy-D-proline, *trans*-3-hydroxy-L-proline and *trans*-3-hydroxy-D-proline.

(11) The agent for improving skin epidermal barrier function according to any one of (7) to (10); wherein the hydroxyproline or hydroxyproline in the *N*-acyl derivative of hydroxyproline is hydroxyproline produced by a microorganism.

(12) The agent for improving skin epidermal barrier function according to (11), wherein the microorganism is a microorganism into which a proline 3-hydroxylase gene or a proline 4-hydroxylase gene derived from a microorganism belonging to a genus selected from the genus *Amycolatopsis*, the genus *Dactylosporangium* and the genus *Streptomyces* is introduced.

(13) An agent for preventing or improving atopic dermatitis which comprises, as an active ingredient, hydroxyproline or an *N*-acyl derivative of hydroxyproline, or a salt thereof.

(14) The agent for preventing or improving atopic dermatitis according to (13), wherein the hydroxyproline or the *N*-acyl derivative of hydroxyproline, or the salt thereof is contained in an amount of 0.01 to 20% by weight based on the total weight.

(15) The agent for preventing or improving atopic dermatitis according to (13) or (14), wherein the *N*-acyl derivative of hydroxyproline is an *N*-acetylated derivative, an *N*-propionylated derivative, an *N*-butyrylated derivative or an isobutyrylated derivative of hydroxyproline.

(16) The agent for preventing or improving atopic dermatitis according to any one of (13) to (15), wherein the hydroxyproline or hydroxyproline in the *N*-acyl derivative of hydroxyproline is an *N*-acyl derivative of hydroxyproline selected from the group consisting of *cis*-4-hydroxy-L-proline, *cis*-4-hydroxy-D-proline, *cis*-3-hydroxy-L-proline, *cis*-3-hydroxy-D-proline, *trans*-4-hydroxy-L-proline, *trans*-4-hydroxy-D-proline, *trans*-3-hydroxy-L-proline and *trans*-3-hydroxy-D-proline.

(17) The agent for preventing or improving atopic dermatitis according to any one of (13) to (16), wherein the hydroxyproline or hydroxyproline in the *N*-acyl derivative of hydroxyproline is hydroxyproline produced by a microorganism.

(18) The agent for preventing or improving atopic dermatitis according to (17), wherein the microorganism is a microorganism into which a proline 3-hydroxylase gene or a proline 4-hydroxylase gene derived from a microorganism belonging to a genus selected from the genus *Amycolatopsis,* the genus *Dactylosporangium* and the genus *Streptomyces* is introduced.

(19) A cosmetic for improving skin barrier function or improving atopic dermatitis, which comprises the ceramide synthesis accelerator according to any one of (1) to (6).

The hydroxyproline used in the present invention has 8 stereoisomers depending on whether the proline is D-form or L-form, whether the position of a hydroxyl group is 3-position or 4-position and whether the stereoisomer is *cis* or *trans*, and any of them can be used.

Examples of the hydroxyproline include *cis*-4-hydroxy-L-proline, *cis*-4-hydroxy-D-proline, *cis*-3-hydroxy-L-proline, *cis*-3-hydroxy-D-proline, *trans*-4-hydroxy-L-proline, *trans*-4-hydroxy-D-proline, *trans*-3-hydroxy-L-proline and *trans*-3-hydroxy-D-proline.

Hydroxyproline is an amino acid which is broadly present in the natural world as an important constituent amino acid component in collagen and as a constituent amino acid of elastin, and it can be produced, e.g., by acid-hydrolyzing collagen derived from an animal such as a pig or bovine and purifying in a conventional way.

*trans*-4-Hydroxy-L-proline can be produced by using a proline 4-hydroxylase (Japanese Published Unexamined Patent Application No. 313179/95) isolated from the genus *Amycolatopsis* or the genus *Dactylosporangium.* Also, the *cis*-3-hydroxy-L-proline can be produced by using a proline 3-hydroxylase (Japanese Published Unexamined Patent Application No. 322885/95) isolated from the genus *Streptomyces* [*Bioindustry*, 14, 31 (1997)].

Specifically, hydroxyproline can be produced by inserting a gene encoding a proline 3-hydroxylase or a proline 4-hydroxylase derived from the above microorganism into an appropriate vector to thereby produce a recombinant vector, introducing the recombinant vector into a microorganism used as the host, and then culturing the microorganism.

In the present invention, hydroxyproline produced by using a microorganism is preferred because a product having more excellent quality can be obtained easily.

Examples of the *N*-acyl derivatives of hydroxyproline used in the present invention include *N*-acyl derivatives of the above stereoisomers of various hydroxyproline. Although the acyl group of the *N*-acyl derivatives is not particularly limited, examples include an acyl group having preferably 1 to 24 carbon atoms, more preferably 1 to 12 carbon atoms, and most preferably 1 to 6 carbon atoms. Specifically, examples of the acyl group include formyl, acetyl, propionyl, butyryl, isobutyryl, valeryl, pivaloyl, hexanoyl, heptanoyl, octanoyl, nonanoyl, decanoyl, undecanoyl, dodecanoyl and the like. Among these, acetyl, propionyl, butyryl or isobutyryl is preferred.

Examples of the salts of the hydroxyproline or the N-acyl derivatives of hydroxyproline include salts with an alkali metal such as sodium, potassium or lithium; salts with an alkaline earth metal such as calcium or magnesium; ammonium salts; addition salts of an amine such as monoethanolamine, diethanolamine, triethanolamine or triisopropanolamine; addition salts of a basic amino acid such as arginine or lysine; and the like.

The *N*-acyl derivatives of hydroxyproline can be produced by a known method. For example, the *N*-acyl derivatives of hydroxyproline can be produced either by converting a straight or branched, saturated or unsaturated fatty acid having 1 to 24 carbon atoms into a halide such as a chloride or a bromide with a halogenating agent such as thionyl chloride or phosgene, and then condensing the halide with the above hydroxyproline; or by converting a fatty acid into an acid anhydride and then reacting the acid anhydride with hydroxyproline.

Examples of the fatty acid include fatty acids such as formic acid, acetic acid, propionic acid, butyric acid, isobutyric acid, valeric acid, isovaleric acid, pivalic acid, hexanoic acid, heptanoic acid, octanoic acid, nonanoic acid, decanoic acid, undecanoic acid and dodecanoic acid, which may be used alone or in combination thereof.

An example of process for producing an *N*-acyl derivative of hydroxyproline via an acid halide is shown below.

A fatty acid is dispersed in a solvent such as methylene chloride, chloroform, carbon tetrachloride, benzene, toluene, xylene or n-hexane, 1 to 5 equivalents of a halogenating agent of 1 to 5 equivalents is added thereto, and the mixture is allowed to react to give a halide of the fatty acid. Next, hydroxyproline is dissolved or dispersed in a solvent, and then keeping the temperature of the solution at 5 to 70°C, the above halide of the fatty acid is added thereto in an amount of 0.3 to 3.0 equivalents based on hydroxyproline to carry out an acylation to give an *N*-acyl derivative of hydroxyproline.

Examples of the solvent used in the acylation include water, methanol, ethanol, isopropanol, isobutanol, acetone, toluene, tetrahydrofuran, ethyl acetate, *N,N*-dimethylformamide, dimethyl sulfoxide and the like, which may be used alone or in combination thereof. When hydroxyproline is dissolved or dispersed in the solvent, an alkaline substance such as sodium hydroxide or potassium hydroxide may be dissolved or dispersed in the solvent in an amount of 0.8 to 2.0 equivalents based on hydroxyproline, if necessary.

In production of a salt of the *N*-acyl derivative of hydroxyproline, when the *N*-acyl derivative of hydroxyproline is obtained in the form of a salt, it may be purified as such, and when it is obtained as a free form, it may be dissolved or dispersed in an appropriate solvent to form a salt by adding a base thereto.

The purification is carried out by using general methods such as crystallization or chromatography.

Specifically, examples of the *N*-acyl derivatives of hydroxyproline include *N*-acetyl-*cis*-4-hydroxy-L-proline, *N*-acetyl-*cis*-4-hydroxy-D-proline, *N*-acetyl-*cis*-3-hydroxy-L-proline, *N*-acetyl-*cis*-3-hydroxy-D-proline, *N*-acetyl-*trans*-4-hydroxy-L-proline, *N*-acetyl-*trans*-4-hydroxy-D-proline, *N*-acetyl-*trans*-3-hydroxy-L-proline, *N*-acetyl-*trans*-3-hydroxy-D-proline, *N*-propionyl-*cis*-4-hydroxy-L-proline, *N*-propionyl-*cis*-4-hydroxy-D-proline, *N*-propionyl-*cis*-3-hydroxy-L-proline, *N*-propionyl-*cis*-3-hydroxy-D-proline, *N*-propionyl-*trans*-4-hydroxy-L-proline, *N*-propionyl-*trans*-4-hydroxy-D-proline, *N*-propionyl-*trans*-3-hydroxy-L-proline, *N*-propionyl-*trans*-3-hydroxy-D-proline, *N*-butyryl-cis-4-hydroxy-L-proline, *N*-butyryl-*cis*-4-hydroxy-D-proline, *N*-butyryl-*cis*-3-hydroxy-L-proline, *N*-butyryl-*cis*-3-hydroxy-D-proline, *N*-butyryl-*trans*-4-hydroxy-L-proline, *N*-butyryl-*trans*-4-hydroxy-D-proline, *N*-butyryl-*trans*-3-hydroxy-L-proline, *N*-butyryl-*trans*-3-hydroxy-D-proline, *N*-isobutyryl-*cis*-4-hydroxy-L-proline, *N*-isobutyryl-*cis*-4-hydroxy-D-proline, *N*-isobutyryl-*cis*-3-hydroxy-L-proline, *N*-isobutyryl-*cis*-3-hydroxy-D-proline, *N*-isobutyryl-*trans*-4-hydroxy-L-proline, *N*-isobutyryl-*trans*-4-hydroxy-D-proline, *N*-isobutyryl-*trans*-3-hydroxy-L-proline, *N*-isobutyryl-trans-3-hydroxy-D-proline and the like.

Examples of the hydroxyproline or the *N*-acyl derivative of hydroxyproline, or the salt thereof in the skin epidermal ceramide synthesis accelerator of the present invention, include *cis/trans*-4-hydroxy-L/D-proline, *cis/trans*-3-hydroxy-L/D-proline or various *N*-acyl derivatives thereof, or salts thereof, which may be used alone or in combination thereof.

The amount of the hydroxyproline or the *N*-acyl derivative of hydroxyproline, or the salt thereof contained in the skin epidermal ceramide synthesis accelerator can be increased or decreased depending on the desired effect, and, for example, the amount is 0.001 to 50% by weight, preferably 0.01 to 20% by weight, and most preferably 0.1 to 10% by weight.

In the present invention, the ceramides mean *N*-acylsphingosine derivatives, and examples thereof include compounds described in Fig. 2 of *Journal of Lipid Research,* 24, 559 (1983) and the like.

The skin epidermis to which the present invention can be applied is not particularly limited to specific ones. Examples of the skin epidermis include the skin epidermis of pets such as a mouse, dog, cat or horse, and that of a human, and human skin epidermis is preferred.

For example, the skin epidermal ceramides can be obtained by extracting epidermal lipid with 95% ethanol, further extracting the extract with hexane : methanol (2 : 3), followed by drying, dissolving the resulting epidermal lipids in chloroform, and separating the skin epidermal ceramides by silica gel thin layer chromatography in a manner similar to the method of Imokawa *et al*. [*Journal of Investigative Dermatology,* 96, 523 (1991)], and measuring the skin epidermal ceramids by using a flying-spot scanning densitometer (manufactured by Shimadzu Corporation, CS-9000).

The skin epidermal ceramide synthesis accelerator of the present invention can be used in cosmetics, medicaments or the like by containing optionally additives suitable for each use, e.g., medicinal carriers or components generally formulated in cosmetics, in addition to the above essential component.

Embodiments of the form of cosmetics and medicaments based on the skin epidermal ceramide synthesis accelerator of the present invention are described below with reference to examples, though not limited thereto.

The form of the cosmetics of the present invention includes liquid products, gel products, emulsion products and solid products such as cream. Examples include face lotion, emulsion, beauty lotion, gel, pack, moisture cream, cold cream, massage cream, after-shaving cream, hand cream, sun protection cream, cleansing cream, body lotion, body shampoo, hair shampoo, face cleansing cream, face cleansing foam, cleansing cream, cleansing milk, cleansing lotion, massage cream, suntan cream, suntan oil, hair rinse, hair treatment, hair tonic, hair care tonic, stick pomade, hair cream, hair liquid, set lotion, hair spray, hair dye, hair bleach, color rinse, color spray, a permanent wave solution, press powder, loose powder, eye shadow, hand cream and the like.

The cosmetics of the present invention may contain general materials used in cosmetics, e.g., solid or semisolid oils, a liquid oil, a moisture keeping agent, an emollient agent, a water-soluble polymer, an oil-soluble polymer, various surfactants, inorganic and organic pigments which may be treated with silicone or a fluorine compound, ethanol, an ultraviolet ray absorbent, an antiseptic, a pH-adjusting agent, a skin softener, water or the like, with hydroxyproline or an N-acyl derivative of hydroxyproline, or a salt thereof. They can be contained within qualitative and quantitative ranges which do not spoil the object and effect of the present invention.

Examples of the solid or semisolid oils include vaseline, lanolin, ceresin, microcrystalline wax, carnauba wax, candelilla wax and beeswax; higher fatty acids such as coconut oil fatty acids, lauric acid and hardened tallow fatty acids; higher alcohols such as lauryl alcohol, cetyl alcohol, stearyl alcohol and behenyl alcohol; and the like.

Examples of the liquid oil include plant oils such as avocado oil, olive oil, jojoba oil and wheat germ oil; fatty acids such as oleic acid and isostearic acid; alcohols such as hexadecyl alcohol and oleyl alcohol; esters such as cetyl 2-ethylhexanoate, 2-octyldodecyl myristate, neopentylglycol di-2-ethylhexanoate, glycerol tri-2-ethylhexanoate, 2-octyldodecyl oleate, isopropyl myristate, glycerol triisostearate, 2-ethylhexanoic acid diglyceride and long chain acylglutamic acid octyldodecyl esters; silicon oil such as dimethyl polysiloxane, methyl hydrogen polysiloxane, methylphenyl polysiloxane and octamethyl cyclotetrasiloxane; liquid hydrocarbon oils such as liquid paraffin, squalene and squalane; and the like.

Examples of the moisture keeping agent include a fat-soluble moisture keeping agent, a low molecular moisture keeping agent and a high molecular moisture keeping agent.

Examples of the fat-soluble moisture keeping agent include lysolecithin, lecithin, cholesterol, cholesterol esters, sphingolipids, ceramides and the like.

Examples of the low molecular moisture keeping agent include serine, glutamine, sorbitol, mannitol, glycerol, sodium pyrrolidonecarboxylate, 1,3-butylene glycol, propylene glycol, lactic acid, lactates and the like.

Examples of the high molecular moisture keeping agent include hyaluronic acid, sodium hyaluronate, elastin, alginic acid, mucopolysaccharides, polyethylene glycol, polyaspartates, water-soluble chitin, attelo collagen and the like.

Examples of the emollient agent include long chain acylglutamic acid cholesteryl esters, cholesteryl hydroxystearate, 12-hydroxystearic acid, stearic acid, rhodinic acid, lanolin fatty acid cholesteryl esters and the like.

Examples of the water-soluble polymer include water-soluble polymers generally used in cosmetics such as carboxymethyl cellulose, methyl cellulose, hydroxymethyl cellulose, polyvinyl alcohol, polyvinyl pyrrolidone, tragacanth gum, carrageenan, dextrin, dextrin fatty acid esters, carboxyvinyl polymers, xanthan gum, gelatin, sodium alginate and gum arabic.

Examples of the oil-soluble polymer include oil-soluble polymers generally used in cosmetics such as a polyvinyl pyrrolidone-eicosene copolymer, a polyvinyl pyrrolidone-hexadecene copolymer, nitrocellulose and high molecular silicone.

Examples of the surfactant include nonionic surfactants such as polyoxyethylene (hereinafter referred to as "POE") cetyl ether, POE stearyl ether, POE oleyl ether, POE stearic acid ester, POE sorbitan monolaurate, glycerol fatty acid esters such as monoglyceryl stearate, polyglycerol fatty acid esters and polyoxyethylene hydrogenated castor oil; cationic surfactants such as benzalkonium chloride, stearyltrimethylammonium chloride, dicetyldimethylammonium chloride and behenyltrimethylammonium chloride; ampholytic surfactants such as 2-cocoyl-*N*-carboxymethyl-*N*-hydroxyethylimidazolinium betaine and amidoacetic acid betaine; and anionic surfactants such as higher alcohol sulfates, higher alcohol ether sulfates, long chain fatty acid alkali metal salts, long chain fatty acid alkaline earth metal salts, long chain fatty acid basic amino acid salts, *N*-long chain acylamino acids and *N*-long chain acylamino acid salts.

Examples of the organic and inorganic pigments include inorganic powders such as silicic acid, silicic anhydride, magnesium silicate, talc, sericite, mica, kaolin, oxide red of iron, clay, bentonite, titanium-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine, chromium oxide, chromium hydroxide, calamine and carbon black, and complexes thereof; organic powders such as polyamides, polyesters, polypropylene, polystyrene, polyurethanes, vinyl resins, urea resins, phenol resins, fluorine resins, silicone resins, acrylic resins, melamine resins, epoxy resins, polycarbonate resins, a divinylbenzene-styrene copolymer, silk powder, cellulose, CI Pigment Yellow and CI Pigment Orange; complex powders of these inorganic powders and organic powders; and the like.

Examples of the organic powders include metal soap such as calcium stearate; alkylphosphoric acid polyvalent metal salts such as zinc cetylphosphate sodium, zinc laurylphosphate and calcium laurylphosphate; acyl amino acid polyvalent metal salts such as calcium *N*-lauroyl-β-alaninate, zinc *N*-lauroyl-β-alaninate and calcium *N*-lauroylglycinate; amidosulfonic acid polyvalent metal salts such as calcium *N*-lauroyltaurinate and calcium *N*-palmitoyltaurinate; *N*-acyl basic amino acids such as *N*ε-lauroyl-L-lysine, *N*ε-palmitoyl lysine, *N*α-palmitoyl ornithine, *N*α-lauroyl arginine and *N*α-hardened tallow fatty acid acylarginine; *N*-acyl polypeptides such as *N*-lauroyl glycylglycine; α-amino fatty acids such as α-aminocapric acid and α-aminolauric acid; resin powders such as polyethylene, polypropylene, nylon, polymethyl methacrylate, polystyrene, a divinylbenzene-styrene copolymer and ethylene tetrafluoride; and the like.

Examples of the ultraviolet ray absorbent include p-oxybenzoic acid derivatives such as ethyl p-oxybenzoate and butyl p-oxybenzoate; p-aminobenzoic acid derivatives such as p-aminobenzoic acid and octyl p-dimethylaminobenzoate; benzophenone derivatives such as 2-hydroxy-4-methoxybenzophenone and dihydroxydimethoxybenzophenone; methoxycinnamic acid derivatives such as ethyl p-methoxycinnamate and octyl p-methoxycinnamate; salicylic acid derivatives such as octyl salicylate and homomenthyl salicylate; α-dehydroamino acid derivatives such as *N*-benzoyl-o-methyl-α-dehydrotyrosine 2-ethylhexyl ester; benzalhydantoin derivatives such as 2-ethylhexyl 4-(3,4-dimethoxyphenyl)methylene-2,5-dioxo-1-imidazolidinepropionate; urocanic acid; ethyl urocanate; 4-*tert*-butyl-4'-methoxydibenzoylmethane; 2-(2'-hydroxy-5'-methylphenyl)benzotriazole; and the like.

Examples of the antiseptic include methylparaben, propylparaben and the like.

Examples of the skin softener include liquid paraffin, vaseline, white vaseline, olive oil, squalane, lanolin, hydrogenated lanolin, synthetic ester oils and the like.

Examples of the pH-adjusting agent include citric acid, sodium citrate and the like.

Any of the above components can be blended within such a range that it does not spoil the object and effect of the present invention, and it is preferably 0.01 to 5% by weight, and more preferably 0.01 to 3% by weight.

The pharmaceutical preparation according to the present invention may comprise, as an active ingredient, hydroxyproline or an N-acyl derivative of hydroxyproline, or a salt thereof alone, or in combination with an ingredient effective for other optional treatment. Also, the pharmaceutical preparation can be made into any dosage form well known in the technical field of pharmaceutics, by mixing the active ingredient together with at least one pharmaceutically acceptable carrier. Examples of the form of the medicaments according to the present invention include ointments, creams, cataplasms, tapes, external preparations and the like.

Examples of the carrier include a binder, a lubricant, a dispersing agent, a suspending agent, an emulsifier, a diluent, a buffering agent, an antioxidant, a bacteria inhibitor and the like.

The hydroxyproline or the *N*-acyl derivative of hydroxyproline, or the salt thereof can be directly administered alone, but it is preferred to provide it generally as various pharmaceutical preparations.

The method for using cosmetics or medicaments of the present invention, which comprises a ceramide synthesis accelerator comprising hydroxyproline or an *N*-acyl derivative of hydroxyproline, or a salt thereof, varies depending on the age, individual and the region to be applied, but it is preferred to apply a cosmetic or medicament which comprises hydroxyproline or an *N*-acyl derivative of hydroxyproline, or a salt thereof having a concentration of 0.001 to 50% by weight, preferably 0.01 to 20% by weight, and more preferably 0.1 to 10% by weight, to the skin at a dose of 0.1 to 5 μl, preferably 1 to 5 μl, and more preferably 2 μl, from once to several times per day, though not limited thereto.

Next, the present invention is described in more detail based on Examples and Test Examples, but the present invention is not limited thereto.

BEST MODE FOR CARRYING OUT THE INVENTION

Example 1: Preparation of face lotion

| Oil phase components: | |
|---|---|
| Perfume (*dl*-rose oxide, manufactured by Kimura Sangyo Co., Ltd.) | 0.05 g |
| Polyoxyethylene(60 mol) hydrogenated castor oil (manufactured by Nihon Emulsion Co., Ltd.) | 2.0 g |
| 1,3-Butylene glycol (manufactured by Kyowa Hakko Kogyo Co., Ltd.) | 5.0 g |
| Aqueous phase components: | |
| *N*-Acetyl-*trans*-4-hydroxy-L-proline (manufactured by Kyowa Hakko Kogyo Co., Ltd.) | 3.0 g |
| Glycerol (manufactured by Kyowa Hakko Kogyo Co., Ltd.) | 5.0 g |
| Methylparaben (manufactured by Ueno Fine Chemicals Industry, Ltd.) | 0.1 g |
| Citric acid (manufactured by Wako Pure Chemical Industries, Ltd.) | 0.1 g |
| Sodium citrate (manufactured by Wako Pure Chemical Industries, Ltd.) | 0.2 g |
| Ethanol (manufactured by Japan Alcohol Trading Co., Ltd.) | 8.0 g |
| Purified water | 100.0 g |

Preparation method:

Each of the oil phase components and the aqueous phase components was homogenized, and then the oil phase was added to the aqueous phase under stirring to give a face lotion.

Example 2: Preparation of emulsion

| Oil phase components: | |
|---|---|
| Squalane (manufactured by Iwase Cosfa Co., Ltd.) | 4.0 g |
| Wheat germ oil (manufactured by Summit Oil Mill Co., Ltd.) | 2.0 g |
| Monoglyceryl stearate (manufactured by Nikko Chemicals Co., Ltd.) | 1.0 g |
| Polyoxyethylene stearyl ether (manufactured by Nihon Emulsion Co., Ltd.) | 4.0 g |
| Propylparaben (manufactured by Ueno Fine Chemicals Industry, Ltd.) | 0.1 g |
| Aqueous phase components: | |
| *N*-Acetyl-*trans*-4-hydroxy-L-proline (manufactured by Kyowa Hakko Kogyo Co., Ltd.) | 3.0 g |
| Methylparaben (manufactured by Ueno Fine Chemicals Industry, Ltd.) | 0.1 g |
| Propylene glycol (manufactured by Wako Pure Chemical Industries, Ltd.) | 0.1 g |
| Polyethylene glycol 6000 (manufactured by Nippon Oil & Fats) | 0.2 g |
| Purified water | 80.5 g |
| 1% Sodium hyaluronate (manufactured by Nikko Chemicals Co., Ltd.) | 5.0 g |

Preparation method:

Each of the oil phase components and the aqueous phase components was homogenized by heating at 80°C, and then the aqueous phase was added to the oil phase under stirring to give an emulsion.

Example 3: Preparation of cream

| Oil phase components: | |
|---|---|
| Squalane (manufactured by Nikko Chemicals Co., Ltd.) | 5.0 g |
| Olive oil (manufactured by Nikko Chemicals Co., Ltd.) | 3.0 g |
| Hydrogenated lanolin (manufactured by Noda Wax Co., Ltd.) | 2.0 g |
| Beeswax (manufactured by Noda Wax Co., Ltd.) | 2.5 g |
| Monoglyceryl stearate (manufactured by Nikko Chemicals Co., Ltd.) | 2.0 g |
| Polyoxyethylene stearyl ether (manufactured by CBC Co., Ltd.) | 2.5 g |
| Propylparaben (manufactured by Ueno Fine Chemicals Industry, Ltd.) | 1.5 g |
| 1,3-Butylene glycol (manufactured by Kyowa Hakko Kogyo Co., Ltd.) | 5.0 g |
| Perfume (*dl*-rose oxide, manufactured by Kimura Sangyo Co., Ltd.) | trace |
| Aqueous phase components: | |
| *N*-Acetyl-*trans*-4-hydroxy-L-proline (manufactured by Kyowa Hakko Kogyo Co., Ltd.) | 5.0 g |
| Methylparaben (manufactured by Ueno Fine Chemicals Industry, Ltd.) | 0.5 g |
| Carbopol 940 (manufactured by Goodrich Corporation) | 0.03 g |
| Triethanolamine (manufactured by Kokusan Chemical Co., Ltd.) | 0.3 g |
| Purified water | 70.97 g |

Preparation method:

Each of the oil phase components and the aqueous phase components was homogenized by heating at 80°C, and then the aqueous phase was added to the oil phase under stirring for emulsification, followed by cooling, to give a cream.

Example 4: Preparation of beauty lotion

| Oil phase components: | |
|---|---|
| Cholesteryl ether (manufactured by Nihon Emulsion Co., Ltd.) | 0.2 g |
| Pyroglutamic acid ether (manufactured by Nihon Emulsion Co., Ltd.) | 1.0 g |
| Lanolin (manufactured by Noda Wax Co., Ltd.) | 0.3 g |
| 1,3-Butylene glycol (manufactured by Kyowa Hakko Kogyo Co., Ltd.) | 5.0 g |
| Perfume (Geraniol, manufactured by Kimura Sangyo Co., Ltd.) | trace |
| Aqueous phase components: | |
| *N*-Acetyl-*trans*-4-hydroxy-L-proline (manufactured by Kyowa Hakko Kogyo Co., Ltd.) | 5.0 g |
| 1% Carbopol (manufactured by CBC Co., Ltd.) | 5.0 g |
| Chondroitin sulfate sodium (manufactured by Iwase Cosfa Co., Ltd.) | 0.02 g |
| Ethanol (manufactured by Japan Alcohol Trading Co., Ltd.) | 1.0 g |
| Methylparaben (manufactured by Ueno Fine Chemicals Industry, Ltd.) | 0.1 g |
| 1% Hyaluronic acid (manufactured by Nikko Chemicals Co., Ltd.) | 8.0 g |
| 0.3% Attelo collagen (manufactured by Koken Co., Ltd.) | 1.0 g |
| Purified water | 73.38 g |

Preparation method:

Each of the oil phase components and the aqueous phase components was homogenized by heating at 80°C, and then the aqueous phase was added to the oil phase under stirring to give a beauty lotion.

Example 5: Preparation of skin powder

| | |
|---|---|
| N-Acetyl-trans-4-hydroxy-L-proline (manufactured by Kyowa Hakko Kogyo Co., Ltd.) | 5.0 g |
| Methylparaben (manufactured by Ueno Fine Chemicals Industry, Ltd.) | 0.5 g |
| Gum arabic (manufactured by Iwase Cosfa Co., Ltd.) | 0.03 g |
| Citric acid (manufactured by Wako Pure Chemical Industries, Ltd.) | 0.3 g |
| Sodium citrate (manufactured by Wako Pure Chemical Industries, Ltd.) | 0.2 g |
| Mannitol (manufactured by Iwase Cosfa Co., Ltd.) | adequate amount |

Preparation method:

The components were homogenized and mixed under stirring to give a powder.

Example 6: Preparation of ointment

| Oil phase components: | |
|---|---|
| White vaseline (manufactured by Iwase Cosfa Co., Ltd.) | 0.2 g |
| Stearyl alcohol (manufactured by Nikko Chemicals Co., Ltd.) | 1.0 g |
| Lauryl sulfate sodium (manufactured by Iwase Cosfa Co., Ltd.) | 0.3 g |
| Aqueous phase components: | |
| N-Acetyl-trans-4-hydroxy-L-proline (manufactured by Kyowa Hakko Kogyo Co., Ltd.) | 5.0 g |
| Propylene glycol (manufactured by Wako Pure Chemical Industries, Ltd.) | 5.0 g |
| Ethyl p-oxybenzoate (manufactured by Ueno Fine Chemicals Industry, Ltd.) | 0.02 g |
| Butyl p-oxybenzoate (manufactured by Ueno Fine Chemicals Industry, Ltd.) | 1.0 g |
| Purified water | 81.45 g |

Preparation method:

Each of the oil phase components and the aqueous phase components was homogenized by heating at 80°C, and then the aqueous phase was added to the oil phase under stirring for emulsification, followed by cooling, to give an ointment.

Example 7: Preparation of pack

| Oil phase components: | |
|---|---|
| Ethanol (manufactured by Japan Alcohol Trading Co., Ltd.) | 8.0 g |
| Polyoxyethylene oleyl ether (manufactured by Nikko Chemicals Co., Ltd.) | 1.0 g |
| Methyl p-oxybenzoate | 0.2 g |
| Aqueous phase components: | |
| N-Acetyl-trans-4-hydroxy-L-proline (manufactured by Kyowa Hakko Kogyo Co., Ltd.) | 5.0 g |
| Propylene glycol | 4.0 g |
| Glycerol (manufactured by Kyowa Hakko Kogyo Co., Ltd.) | 5.0 g |
| Polyvinyl alcohol (manufactured by Shin-Etsu Chemical Co., Ltd.) | 15.0 g |
| Purified water | 61.6 g |
| Perfume (Geraniol, manufactured by Kimura Sangyo Co., Ltd.) | 0.2 g |

Preparation method:

Each of the aqueous phase components and the oil phase components was homogenized by heating at 80°C, and then the aqueous phase was added to the oil phase under stirring to give a pack.

Example 8: Preparation of tapes

| Adhesive solvent: | |
|---|---|
| Styrene-isopropylene-styrene block copolymer (manufactured by Shell Japan Ltd.) | 7.0 g |
| Ester gum (manufactured by Dainippon Ink & Chemicals, Inc.) | 25.0 g |
| Isopropylene gum (manufactured by Kuraray Co., Ltd.) | 5.0 g |
| Toluene (manufactured by Iwase Cosfa Co., Ltd.) | 15.0 g |
| Ethyl acetate (manufactured by Kishida Chemical Co., Ltd.) | 14.2 g |
| Hexane (manufactured by Kishida Chemical Co., Ltd.) | 25.0 g |
| Pharmaceutically active components: | |
| N-Acetyl-trans-4-hydroxy-L-proline (manufactured by Kyowa Hakko Kogyo Co., Ltd.) | 3.0 g |
| Ethanol (manufactured by Japan Alcohol Trading Co., Ltd.) | 5.0 g |
| Percutaneous absorption accelerator: | |
| Oleyl alcohol (manufactured by Iwase Cosfa Co., Ltd.) | 0.8 g |

Preparation method:

Each of the adhesive solvent and the pharmaceutically active components was homogenized, and then the pharmaceutically active components and the percutaneous absorption accelerator were added to the adhesive solvent under stirring at room temperature to give a composition. The composition was spread on a silicone-treated polyester film, dried at 120°C and cooled, and then the adhesive layer was transferred on a polyethylene film to give a tape.

Test Example 1

Evaluation of the amount of epidermal ceramide synthesis in hairless mice:

Test Compositions 1 to 5 were obtained by preparing 30% by weight aqueous ethanol solutions containing 0, 1.0, 3.0, 5.0 and 10.0% by weight, respectively, of N-acetyl-trans-4-hydroxy-L-proline. The pH values of the aqueous solutions were adjusted to 4.5 with sodium hydroxide.
Each of Test Compositions 1 to 5 was applied at 200 $\mu$l to the entire dorsal area as the tested region of each hairless mouse (SKH1:hr/hr:BR male, 7 week old, available from Charles River Japan, Inc.) once a day for one month. The mice were used as four animals per each group.
Before starting of the application and one month after starting of the application, epidermal lipids were extracted from the entire dorsal area as the tested region of each mouse, and the activity of N-acetyl-trans-4-hydroxy-L-proline to synthesize ceramide was evaluated by measuring the ceramide content.
The epidermal lipids were obtained by extraction with 95% ethanol and further extracting the extract with hexane : methanol (2 : 3), followed by drying. The resulting epidermal lipids were dissolved in chloroform and separated by using silica gel thin layer chromatography in a manner similar to the method of Imokawa et al. [*Journal of Investigative Dermatology,* 96, 523 (1991)], and then the ceramide content was measured using a flying-spot scanning densitometer (manufactured by Shimadzu Corporation, CS-9000).
The ceramide content was calculated in accordance with the following equation, as the relative value to the change in the amount of ceramide synthesis in untreated mice.

Relative amount of ceramide synthesis (%)

$$= [(A1 \times B2)/(A2 \times B1)] \times 100$$

A1: Ceramide content after application in tested mice
A2: Ceramide content before application in tested mice
B1: Ceramide content after application in untreated mice
B2: Ceramide content before application in untreated mice

Since it is known that the ceramides exist in several isomer forms, the calculation was carried out on each of the amount of total ceramide synthesis and those of syntheses of various ceramide isomers. As the standards, ceramide III (consisting of ceramides 1 and 2) and ceramide IV (consisting of ceramides 4 and 5) both manufactured by Sigma were used. Here, total ceramide means the total of ceramides 1, 2, 4 and 5, and ceramide III means the total of ceramides 1 and 2. The ceramides 1, 2, 4 and 5 correspond to the compounds 1, 2, 4 and 5, respectively, described in Fig. 2 of *Journal of Lipid Research,* 24, 559 (1983).

The results are shown in Tables 1 and 2.

Table 1

| N-acetyl-*trans*-4-hydroxy-L-proline concentration (% by weight) | Relative amount of total ceramide synthesis (%) |
| --- | --- |
| 0 | 111.1 |
| 1 | 218.6 |
| 3 | 170.6 |

Table 2

| N-acetyl-*trans*-4-hydroxy-L-proline concentration (% by weight) | Relative amount of ceramide III synthesis (%) |
| --- | --- |
| 0 | 126.4 |
| 1 | 239.5 |
| 3 | 321.1 |
| 5 | 209.6 |
| 10 | 185.9 |

As a result of the long period of continuous use, significant increase in the total ceramide content in the epidermal stratum corneum was observed at an N-acetyl-*trans*-4-hydroxy-L-proline concentration of 1 to 3% by weight, and the significant increase in ceramide III was observed at an N-acetyl-*trans*-4-hydroxy-L-proline concentration of 1 to 10% by weight.

Thus, since the ceramide synthesis accelerator of the present invention has a function to accelerate ceramide synthesis in the epidermal stratum corneum, it is effective in improving skin diseases such as chapped skin or atopic dermatitis through the improvement of the skin barrier function.

Test Example 2

Evaluation of auricular edema inhibition in atopic dermatitis-induced model mice (type I allergy model):

Test Compositions 6 to 8 were obtained by preparing 30% by weight aqueous ethanol solutions containing 0, 1.0 and 3.0% by weight, respectively, of N-acetyl-*trans*-4-hydroxy-L-proline. The pH values of the aqueous solutions were adjusted to 4.5 with sodium hydroxide.

Atopic dermatitis using NC/Nga mice was carried out in a same manner similar to the method of Sasagawa et al. [16th Japanese Society of Disease Models (1999)]. Auricular edema was induced by intradermal injection of 20 μg of a tick (*Dermatophagoides pteronyssinus*) extract as the antigen into the auricle of NC/Nga mouse (male, 8 week old, available from Charles River Japan, Inc.), once a day at a predetermined time in the 1st day, 3rd day and every day from 7th days after starting of the test. Each of Test Compositions 6 to 8 was applied at 20 μl to both sides of the auricle as the tested region, 6 hours after administration of the tick extract, once a day from the 7th day after starting of the test. The mice were used as six animals per each group.

A thickness of the auricle was measured on the 28th day after starting of the test using a dial thickness gauge (G-1A, manufactured by PEACOCK), and the relative auricular edema increase (%) was calculated in accordance with the following equation.

Relative auricular edema increase (%)

$$= [(A1 \times B2)/(A2 \times B1)] \times 100$$

A1:    Thickness of auricle after passed days in tested mice
A2:    Thickness of auricle at the time of starting of the test in tested mice
B1:    Thickness of auricle after passed days in untreated mice
B2:    Thickness of auricle at the time of starting of the test in untreated mice

The results are shown in Table 3. Each of the data shows average value ± standard deviation (n = 6) of the relative auricular edema increase (%).

Table 3

| N-Acetyl-trans-4-hydroxy-L-proline concentration (% by weight) | Relative auricular edema increase (%) |
|---|---|
| 0 | 133.9 ± 6.1 |
| 1 | 120.9 ± 3.5 |
| 3 | 110.1 ± 4.4 |

As a result of the long period of continuous use, N-acetyl-trans-4-hydroxy-L-proline significantly inhibited auricular edema caused by the type I allergy model at a concentration of 1 to 3% by weight.

Test Example 3

Evaluation of auricular edema inhibition in atopic dermatitis-induced model mice (type I and type IV allergy models):

Test Compositions 9 and 10 were obtained by preparing 30% by weight ethanol solutions containing 0 and 3.0% by weight, respectively, of N-acetyl-trans-4-hydroxy-L-proline. The pH values of the aqueous solutions were adjusted to 4.5 with sodium hydroxide.
Atopic dermatitis using NC/Nga mice was carried out in a manner similar to the method of Fujii et al. [*Fundamentals and Clinics (Kiso-to-Rinsho),* 31(8), 2693 (1997)]. Auricular edema was induced by applying 20 μl of 1.5% dinitrochlorobenzene (DNCB) to both sides of the auricle of NC/Nga mouse (male, 8 week old, available from Charles River Japan, Inc.), once a day at a predetermined time in the 1st, 3rd and 7th days after starting of the test. Each of Test Compositions 9 and 10 was applied at 20 μl to both sides of the auricle as the tested region, 6 hours after the application of DNCB and once a day every day from the 1st day after starting of the test. The mice were used as six animals per each group.
Just before the application of the test compositions to the auricle on the 11th, 12th, 14th and 17th days after starting of the test, a thickness of the auricle was measured using a dial thickness gauge (G-1A, manufactured by PEACOCK), and the relative auricular edema increase (%) was calculated in accordance with the following equation.

Relative auricular edema increase (%)

$$= [(A1 \times B2)/(A2 \times B1)] \times 100$$

A1:    Thickness of auricle after passed days in tested mice
A2:    Thickness of auricle at the time of starting of the test in tested mice
B1:    Thickness of auricle after passed days in untreated mice
B2:    Thickness of auricle at the time of starting of the test in untreated mice

The results are shown in Table 4. Each of the data shows average value ± standard deviation (n = 6) of the relative auricular edema increase (%).

Table 4

| Days after starting of test | N-Acetyl-trans-4-hydroxy-L-proline concentration | |
|---|---|---|
| | 0% by weight | 3% by weight |
| 11th | 540.2 ± 32.2 | 360.1 ± 64.2 |
| 12th | 635.0 ± 53.1 | 399.7 ± 55.9 |
| 14th | 699.2 ± 29.2 | 429.0 ± 72.3 |
| 17th | 494.7 ± 65.9 | 312.1 ± 50.6 |

As a result of the long period of continuous use, N-acetyl-trans-4-hydroxy-L-proline significantly inhibited auricular edema caused by the type I and type IV allergy models at a concentration of 3% by weight.

INDUSTRIAL APPLICABILITY

The present invention provides skin epidermal ceramide synthesis accelerators comprising, as an active ingredient, hydroxyproline or an N-acyl derivative of hydroxyproline, or a salt thereof, which increases ceramide biosynthesis capacity of the skin and is effective in improving skin diseases such as chapped skin or atopic dermatitis.

**Claims**

1.  A skin epidermal ceramide synthesis accelerator which comprises, as an active ingredient, hydroxyproline or an N-acyl derivative of hydroxyproline, or a salt thereof.

2.  The skin epidermal ceramide synthesis accelerator according to claim 1, wherein the hydroxyproline or the N-acyl derivative of hydroxyproline, or the salt thereof is contained in an amount of 0.01 to 20% by weight based on the total weight.

3.  The skin epidermal ceramide synthesis accelerator according to claim 1 or 2, wherein the N-acyl derivative of hydroxyproline is an N-acetylated derivative, an N-propionylated derivative, an N-butyrylated derivative or an isobutyrylated derivative of hydroxyproline.

4.  The skin epidermal ceramide synthesis accelerator according to any one of claims 1 to 3, wherein the hydroxyproline or hydroxyproline in the N-acyl derivative of hydroxyproline is an N-acyl derivative of hydroxyproline selected from the group consisting of cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.

5.  The skin epidermal ceramide synthesis accelerator according to any one of claims 1 to 4, wherein the hydroxyproline or hydroxyproline in the N-acyl derivative of hydroxyproline is hydroxyproline produced by a microorganism.

6.  The skin epidermal ceramide synthesis accelerator according to claim 5, wherein the microorganism is a microorganism into which a proline 3-hydroxylase gene or a proline 4-hydroxylase gene derived from a microorganism belonging to a genus selected from the genus Amycolatopsis, the genus Dactylosporangium and the genus Streptomyces is introduced.

7.  An agent for improving skin epidermal barrier function which comprises, as an active ingredient, hydroxyproline or an N-acyl derivative of hydroxyproline, or a salt thereof.

8.  The agent for improving skin epidermal barrier function according to claim 7, wherein the hydroxyproline or the N-acyl derivative of hydroxyproline, or the salt thereof is contained in an amount of 0.01 to 20% by weight based on the total weight.

9.  The agent for improving skin epidermal barrier function according to claim 7 or 8, wherein the N-acyl derivative of hydroxyproline is an N-acetylated derivative, an N-propionylated derivative, an N-butyrylated derivative or an isobutyrylated derivative of hydroxyproline.

10. The agent for improving skin epidermal barrier function according to any one of claims 7 to 9, wherein the hydroxyproline or hydroxyproline in the N-acyl derivative of hydroxyproline is an *N*-acyl derivative of hydroxyproline selected from the group consisting of *cis*-4-hydroxy-L-proline, *cis*-4-hydroxy-D-proline, *cis*-3-hydroxy-L-proline, *cis*-3-hydroxy-D-proline, *trans*-4-hydroxy-L-proline, *trans*-4-hydroxy-D-proline, *trans*-3-hydroxy-L-proline and *trans*-3-hydroxy-D-proline.

11. The agent for improving skin epidermal barrier function according to any one of claims 7 to 10, wherein the hydroxyproline or hydroxyproline in the *N*-acyl derivative of hydroxyproline is hydroxyproline produced by a microorganism.

12. The agent for improving skin epidermal barrier function according to claim 11, wherein the microorganism is a microorganism into which a proline 3-hydroxylase gene or a proline 4-hydroxylase gene derived from a microorganism belonging to a genus selected from the genus *Amycolatopsis,* the genus *Dactylosporangium* and the genus *Streptomyces* is introduced.

13. An agent for preventing or improving atopic dermatitis which comprises, as an active ingredient, hydroxyproline or an *N*-acyl derivative of hydroxyproline, or a salt thereof.

14. The agent for preventing or improving atopic dermatitis according to claim 13, wherein the hydroxyproline or the *N*-acyl derivative of hydroxyproline, or the salt thereof is contained in an amount of 0.01 to 20% by weight based on the total weight.

15. The agent for preventing or improving atopic dermatitis according to claim 13 or 14, wherein the N-acyl derivative of hydroxyproline is an *N*-acetylated derivative, an *N*-propionylated derivative, an *N*-butyrylated derivative or an isobutyrylated derivative of hydroxyproline.

16. The agent for preventing or improving atopic dermatitis according to any one of claims 13 to 15, wherein the hydroxyproline or hydroxyproline in the *N*-acyl derivative of hydroxyproline is an *N*-acyl derivative of hydroxyproline selected from the group consisting of *cis*-4-hydroxy-L-proline, *cis*-4-hydroxy-D-proline, *cis*-3-hydroxy-L-proline, *cis*-3-hydroxy-D-proline, *trans*-4-hydroxy-L-proline, *trans*-4-hydroxy-D-proline, *trans*-3-hydroxy-L-proline and *trans*-3-hydroxy-D-proline.

17. The agent for preventing or improving atopic dermatitis according to any one of claims 13 to 16, wherein the hydroxyproline or hydroxyproline in the *N*-acyl derivative of hydroxyproline is hydroxyproline produced by a microorganism.

18. The agent for preventing or improving atopic dermatitis according to claim 17, wherein the microorganism is a microorganism into which a proline 3-hydroxylase gene or a proline 4-hydroxylase gene derived from a microorganism belonging to a genus selected from the genus *Amycolatopsis,* the genus *Dactylosporangium* and the genus *Streptomyces* is introduced.

19. A cosmetic for improving skin barrier function or improving atopic dermatitis, which comprises the ceramide synthesis accelerator according to any one of claims 1 to 6.

## INTERNATIONAL SEARCH REPORT

| International application No. |
|---|
| PCT/JP01/06269 |

A. CLASSIFICATION OF SUBJECT MATTER
Int.Cl$^7$   C07D207/16, A61K31/401, A61P43/00, A61P17/00, A61K7/00, A61K7/48

According to International Patent Classification (IPC) or to both national classification and IPC

B.   FIELDS SEARCHED

Minimum documentation searched (classification system followed by classification symbols)
Int.Cl$^7$   C07D207/16, A61K31/401, A61P43/00, A61P17/00, A61K7/00, A61K7/48

Documentation searched other than minimum documentation to the extent that such documents are included in the fields searched

Electronic data base consulted during the international search (name of data base and, where practicable, search terms used)
CA (STN), REGISTRY (STN), WPIDS (STN)

C.   DOCUMENTS CONSIDERED TO BE RELEVANT

| Category* | Citation of document, with indication, where appropriate, of the relevant passages | Relevant to claim No. |
|---|---|---|
| X | JP 11-139951 A (Lion Corporation), 25 May, 1999 (25.05.99)   (Family: none) | 1-19 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 August, 2001 (16.08.01) | 28 August, 2001 (28.08.01) |

| Name and mailing address of the ISA/ | Authorized officer |
|---|---|
| Japanese Patent Office | |
| Facsimile No. | Telephone No. |

Form PCT/ISA/210 (second sheet) (July 1992)